(19) Europäisches Patentamt
European Patent Office
Office européen des brevets

(11) Publication number: **0 163 240**
**A2**

# (12) EUROPEAN PATENT APPLICATION

(21) Application number: 85106208.3

(22) Date of filing: 21.05.85

(51) Int. Cl.[4]: C 07 D 487/04
A 61 K 31/505
//(C07D487/04, 239:00, 235:00)

(30) Priority: 22.05.84 JP 104257/84

(43) Date of publication of application:
04.12.85 Bulletin 85/49

(84) Designated Contracting States:
AT BE CH DE FR GB IT LI NL SE

(71) Applicant: Yoshitomi Pharmaceutical Industries, Ltd.
35 Hiranomachi 3-chome Higashi-ku
Osaka-shi Osaka 541(JP)

(72) Inventor: Tsuda, Yoshinao
2884-24 Higashisayamagaoka 3-chome
Tokorozawa Saitama(JP)

(72) Inventor: Mishina, Tadashi
4-17-601 Ohgimaciya 1-chome
Iruma Saitama(JP)

(72) Inventor: Obata, Minoru
4-36, Okidaimachi 1-chome
Nakatsu Oita(JP)

(72) Inventor: Inui, Jun
1129-78, Noda
Iruma Saitama(JP)

(74) Representative: von Kreisler, Alek, Dipl.-Chem. et al,
Deichmannhaus am Hauptbahnhof
D-5000 Köln 1(DE)

## (54) Dihydroimidazo1,2-apyrimidine derivatives, methods of preparing said compounds and pharmaceutical compositions constaining said compounds.

(57) A dihydroimidazo [1, 2-a] pyrimidine derivative of the formula:

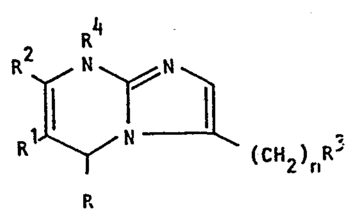

wherein R is hydrogen, $C_{1-5}$ alkyl, heteroaryl, 2, 1, 3-benzoxadiazolyl or phenyl which may be substituted by a substituent selected from halogen, $C_{1-4}$ alkyl, trifluoromethyl, $C_{1-4}$ alkoxy, nitro and cyano;

$R^1$ is cyano, $C_{1-4}$ alkoxy-carbonyl or $-COOCH(R^5)CH_2R^6$ [wherein $R^5$ is hydrogen or aryl and $R^6$ is $C_{1-4}$ alkoxy or $-N(Ra)(Rb)$ (wherein each of Ra and Rb is $C_{1-4}$ alkyl or aralkyl, or Ra and Rb together with the adjacent nitrogen atom form a heterocycle)];

$R^2$ is $C_{1-4}$ alkyl, hydroxymethyl, acetoxymethyl, di-$C_{1-4}$ alkoxy-methyl, $C_{1-4}$ alkyl-carbamoyloxymethyl or hydroxyimino-methyl;

$R^3$ is $C_{1-4}$ alkyl, formyl, carboxyl, nitro, $-N(R^7)(R^8)$ (wherein each of $R^7$ and $R^8$ is hydrogen, $C_{1-8}$ alkyl, aryl, aralkyl or acyl, or $R^7$ and $R^8$ together with the adjacent nitrogen atom form a heterocycle), $-OR^9$ or $-SR^{10}$ (wherein each of $R^9$ and $R^{10}$ is hydrogen, $C_{1-8}$ alkyl, aralkyl or acyl);

$R^4$ is hydrogen or $-(CH_2)_mN(Rc)(Rd)$ (wherein each of Rc and Rd is $C_{1-4}$ alkyl or aralkyl, or Rc and Rd together with the adjacent nitrogen atom form a heterocycle and m is 1 to 3;

n is 0 or 1 to 3,

inclusive of an optical isomer thereof, a diastereomer thereof and compounds selected from the group consisting of pharmaceutically acceptable acid addition salt forms thereof, hydrate forms thereof and mixtures thereof, methods of preparing said compounds and pharmaceutical compositions containing said compounds are disclosed.

The dihydroimidazo [1, 2-a] pyrimidine derivatives are useful as drugs for the treatment of coronary and cerebral circulatory diseases.

EP 0 163 240 A2

0163240

# DIHYDROIMIDAZO[1,2-a]PYRIMIDINE DERIVATIVES, METHODS OF PREPARING SAID COMPOUNDS AND PHARMACEUTICAL COMPOSITIONS CONTAINING SAID COMPOUNDS

## Field of the Invention

The present invention relates to novel and pharmaceutically useful dihydroimidazo[1,2-a]pyrimidine derivatives, methods of preparing said compounds and pharmaceutical compositions containing said compounds.

## Description of the Prior Art

It is known that 1,4-dihydropyridine compounds exhibit calcium antagonistic activity and are useful for the treatment of circulatory diseases such as angina pectoris, hypertension or cerebral circulatory disorder. In particular, nifedipine (U. S. Patent No. 3485847) and nicardipine (U. S. Patent No. 3985758) have been widely used as calcium antagonist in clinical field.

Certain fused 1,4-dihydropyridine compounds also have calcium antagonistic activity, such as 4,7-dihydropyrazolo [3,4-b]pyridine-5-carboxylic acid derivatives disclosed in European Patent Application Publications Nos. 107619 and 114273.

Further, European Patent Application Publication No. 103796 discloses 1,4-dihydropyrimidine-5-carboxylic acid derivatives having cardiovascular activity, and Japanese Patent Application laid open under No. 95289/84 (Derwent CPI

No. 84-173756) published on June 1, 1984 discloses 1,2,4-triazolo[1,5-a]pyrimidine compounds with calcium antagonistic activity.

Moreover, in our pending application PCT/JP85/00115, there are disclosed polyazaheterocycle derivatives of the formula:

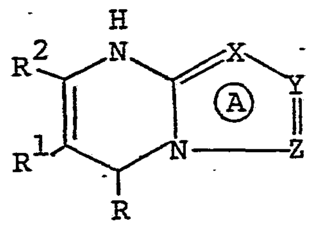

wherein the 5-membered ring (A) has no substituents, inclusive of 4,7-dihydrotetrazolo[1,5-a]pyrimidine compounds, 4,7-dihydrotriazolo[1,5-a]pyrimidine compounds, 5,8-dihydroimidazo[1,2-a]pyrimidine compounds, 1,4-dihydroimidazo[1,5-a]pyrimidine compounds and 4,7-dihydropyrazolo[1,5-a]pyrimidine compounds. These compounds have antihypertensive activity, coronary vasodilating activity, cerebrovasodilating activity, cardiotonic activity and lipid peroxidation inhibiting activity.

Recently, several reports suggest that acetylcholine (ACh) may be a possible spasmogen in human coronary artery (Circulation Research, vol. 55, p. 416-421, 1985). In fact, intra-arterial administration of ACh to patients with angina pectoris results in the attack with coronary spasm and chest pain (Proceedings of 49th annual scientific meeting of the Japanese Circulation Society, 1985). Therefore, the calcium antagonist with anticholinergic activity is thought to be more suitable and reasonable than usual calcium antagonists.

## Summary of the Invention

One purpose of the present invention is to provide novel and pharmaceutically useful dihydroimidazo[1,2-a]pyrimidine derivatives inclusive of compounds selected from the group consisting of pharmaceutically acceptable acid addition salt forms thereof, hydrate forms thereof and mixtures thereof.

Another purpose of the present invention is to provide methods of preparing the dihydroimidazo[1,2-a]pyrimidine derivatives.

Other purpose of the present invention is to provide pharmaceutical compositions containing the dihydroimidazo [1,2-a]pyrimidine derivatives.

## Detailed Description of the Invention

The dihydroimidazo[1,2-a]pyrimidine derivatives of the present invention are represented by the following general formula:

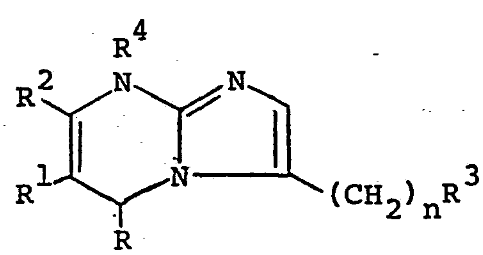

(I)

In the above formula, each symbol is as defined below:

R is a hydrogen atom, a $C_{1-5}$ alkyl group (e.g. methyl, ethyl, propyl, isopropyl, butyl, isobutyl, sec-butyl, tert-butyl or pentyl), a heteroaryl group (e.g. a furyl group, a thienyl group or a pyridyl group), a 2,1,3-benzoxadiazolyl

group or a phenyl group which may be optionally substituted by a substituent selected from the group consisting of a halogen atom (fluorine, chlorine, bromine or iodine), a $C_{1-4}$ alkyl group (e.g. methyl, ethyl, propyl, isopropyl or butyl), a trifluoromethyl group, a $C_{1-4}$ alkoxy group (e.g. methoxy, ethoxy, propoxy or butoxy), a nitro group and a cyano group;

$R^1$ is a cyano group, a $C_{1-4}$ alkoxy-carbonyl group (e.g. methoxycarbonyl, ethoxycarbonyl, propoxycarbonyl or butoxycarbonyl), or a group represented by the formula of $-COOCH(R^5)CH_2R^6$ [wherein $R^5$ is a hydrogen atom or an aryl group (e.g. phenyl or naphthyl) which may be optionally substituted by a substituent selected from the group consisting of a halogen atom such as chlorine or bromine, a $C_{1-4}$ alkyl group such as methyl, ethyl or propyl, a $C_{1-4}$ alkoxy group such as methoxy or ethoxy, a trifluoromethyl group, a nitro group and an amino group, $R^6$ is a $C_{1-4}$ alkoxy group (e.g. methoxy, ethoxy, propoxy or isopropoxy), or a group represented by the formula of $-N(Ra)(Rb)$ (wherein each of Ra and Rb is a $C_{1-4}$ alkyl group (e.g. methyl, ethyl, propyl, isopropyl or butyl) or an aralkyl group (e.g. benzyl, phenylethyl or phenylpropyl), or Ra and Rb together with the adjacent nitrogen atom form a heterocycle), which includes, for example, dimethylamino, diethylamino, dipropylamino, diisopropylamino, N-methyl-N-benzylamino, 1-pyrrolidyl, piperidino, morpholino, 1-piperazinyl, 4-methyl-1-piperazinyl, hexamethyleneimino or imidazolyl];

$R^2$ is a $C_{1-5}$ alkyl group (e.g. methyl, ethyl, propyl, isopropyl, butyl, isobutyl, sec-butyl, tert-butyl or pentyl), a hydroxymethyl group, an acetoxymethyl group, a di-$C_{1-4}$

alkoxy-methyl group (e.g. dimethoxymethyl or diethoxymethyl), a $C_{1-4}$ alkyl-carbamoyloxymethyl group (e.g. methylcarbamoyloxymethyl or ethylcarbamoyloxymethyl) or a hydroxyiminomethyl group;

$R^3$ is a $C_{1-5}$ alkyl group (e.g. methyl, ethyl, propyl, isopropyl, butyl, isobutyl, sec-butyl, tert-butyl or pentyl), a formyl group, a carboxyl group, a nitro group, or a group represented by the formula of $-N(R^7)(R^8)$ [wherein each of $R^7$ and $R^8$ is a hydrogen atom, a $C_{1-8}$ alkyl group (e.g. methyl, ethyl, propyl, isopropyl, butyl, isobutyl, tert-butyl, pentyl, hexyl or octyl), an aryl group (e.g. phenyl or naphthyl) which may be optionally substituted by a substituent selected from the group consisting of a halogen atom such as chlorine or bromine, a $C_{1-4}$ alkyl group such as methyl, ethyl or propyl, a $C_{1-4}$ alkoxy group such as methoxy or ethoxy, a trifluoromethyl group, a nitro group and an amino group, an aralkyl group (e.g. benzyl, phenylethyl or phenylpropyl) which may be optionally substituted on the benzene ring by a substituent selected from the group consisting of a halogen atom such as chlorine or bromine, a $C_{1-4}$ alkyl group such as methyl, ethyl or propyl, a $C_{1-4}$ alkoxy group such as methoxy or ethoxy, a trifluoromethyl group, a nitro group and an amino group, or an acyl group (e.g. acetyl, propionyl, butyryl or benzoyl), or $R^7$ and $R^8$ together with the adjacent nitrogen atom form a 5- or 6-membered heterocycle which may optionally contain an oxygen atom, a sulfur atom or $=NR^{11}$ (wherein $R^{11}$ is a hydrogen atom, a $C_{1-4}$ alkyl group such as methyl or ethyl, a hydroxy-$C_{1-4}$ alkyl group such as hydroxymethyl, hydroxyethyl, hydroxypropyl

or hydroxybutyl, an acyl group such as acetyl, propionyl, butyryl, benzoyl or 2-furylcarbonyl, or a phenyl group which may be optionally substituted by a substituent selected from the group consisting of a halogen atom (e.g. chlorine or bromine), a $C_{1-4}$ alkyl group (e.g. methyl, ethyl, propyl, isopropyl or butyl) and a $C_{1-4}$ alkoxy group (e.g. methoxy, ethoxy, propoxy or butoxy)), which includes, for example, 1-pyrrolidinyl, piperidino, morpholino, thiomorpholino, 1-piperazinyl, 4-methyl-1-piperazinyl, 4-(2-hydroxyethyl)-1-piperazinyl, 4-butyryl-1-piperazinyl, 4-(2-furylcarbonyl)-1-piperazinyl, 4-(2-methylphenyl)-1-piperazinyl or imidazolyl], $-OR^9$ or $-SR^{10}$ [wherein each of $R^9$ and $R^{10}$ is a hydrogen atom, a $C_{1-8}$ alkyl group (e.g. methyl, ethyl, propyl, isopropyl, butyl, isobutyl, sec-butyl, tert-butyl, pentyl, hexyl or octyl), an aralkyl group (e.g. benzyl, phenylethyl or phenylpropyl) which may be optionally substituted on the benzene ring by a substituent selected from the group consisting of a halogen atom such as chlorine or bromine, a $C_{1-4}$ alkyl group such as methyl, ethyl or propyl, a $C_{1-4}$ alkoxy group such as methoxy or ethoxy, a trifluoromethyl group, a nitro group and an amino group, or an acyl group (e.g. acetyl, propionyl, butyryl or benzoyl)];

$R^4$ is a hydrogen atom or a group represented by the formula: $-(CH_2)_m(Rc)(Rd)$ [wherein each of Rc and Rd is a $C_{1-4}$ alkyl group (e.g. methyl, ethyl or propyl) or an aralkyl group (e.g. benzyl, phenylethyl or phenylpropyl) which may be optionally substituted on the benzene ring by a substituent selected from the group consisting of a halogen atom such as

0163240

chlorine or bromine, a $C_{1-4}$ alkyl group such as methyl, ethyl or propyl, a $C_{1-4}$ alkoxy group such as methoxy or ethoxy, a trifluoromethyl group, a nitro group and an amino group, or Rc and Rd together with the adjacent nitrogen atom form a heterocycle (e.g. 1-pyrrolidinyl, piperidino, morpholino, 1-piperazinyl or 4-methyl-1-piperazinyl), and m is an integer of from 1 to 3]; and

n is 0 or an integer of from 1 to 3.

Preferable compounds of the present invention are the compounds of formula (I) wherein R is a hydrogen atom, a $C_{1-5}$ alkyl group, a thienyl group, a 2,1,3-benzoxadiazolyl group or a phenyl group which may be optionally substituted by a substituent selected from the group consisting of a halogen atom, a $C_{1-4}$ alkyl group, a trifluoromethyl group, a $C_{1-4}$ alkoxy group, a nitro group and a cyano group; $R^1$ is a cyano group, a $C_{1-4}$ alkoxy-carbonyl group or a group represented by the formula: $-COOCH(R^5)CH_2R^6$ [wherein $R^5$ is a hydrogen atom or a phenyl group and $R^6$ is a $C_{1-4}$ alkoxy group or a group represented by the formula of -N(Ra)(Rb) (wherein each of Ra and Rb is a $C_{1-4}$ alkyl group, or Ra and Rb together with the adjacent nitrogen atom form a piperidino group)]; $R^2$ is a $C_{1-4}$ alkyl group, a hydroxymethyl group, an acetoxymethyl group, a di-$C_{1-4}$ alkoxy-methyl group, a $C_{1-4}$ alkyl-carbamoyloxy-methyl group or a hydroxyiminomethyl group; $R^3$ is a $C_{1-4}$ alkyl group, a formyl group, a carboxyl group, a nitro group, or a group represented by the formula of $-N(R^7)(R^8)$ (wherein each of $R^7$ and $R^8$ is a hydrogen atom, a $C_{1-8}$ alkyl group, a trifluoromethylphenyl group, a benzyl group or an acyl group,

or $R^7$ and $R^8$ together with the adjacent nitrogen atom form a heterocycle selected from the group consisting of 1-pyrrolidinyl, piperidino, morpholino, 1-piperazinyl, 4-butyryl-1-piperazynyl, 4-(2-furylcarbonyl)-1-piperazinyl and imidazolyl groups), $-OR^9$ (wherein $R^9$ is a hydrogen atom, a $C_{1-4}$ alkyl group, a benzyl group or an acyl group) or $-SR^{10}$ wherein $R^{10}$ is a $C_{1-4}$ alkyl group); $R^4$ is a hydrogen atom or a 2-morpholinoethyl group; and n is 0 or an integer of from 1 to 3, inclusive of compounds selected from the group consisting of pharmaceutically acceptable acid addition salt forms thereof, hydrate forms thereof and mixtures thereof.

More preferable compounds of the present invention are the compounds of formula (I) wherein $R^1$ is a phenyl group substituted by a nitro group or a trifluoromethyl group; $R^1$ is a $C_{1-4}$ alkoxy-carbonyl group or a group represented by the formula of $-COOCH(R^5)CH_2R^6$ [wherein $R^5$ is a hydrogen atom or a phenyl group and $R^6$ is a methoxy group or a group represented by the formula: -N(Ra)(Rb) (wherein Ra and Rb together with the adjacent nitrogen atom form a piperidino group)]; $R^2$ is a $C_{1-4}$ alkyl group; $R^3$ is a nitro group or a group represented by the formula of $-N(R^7)(R^8)$ (wherein each of $R^7$ and $R^8$ is a $C_{1-4}$ alkyl group or a benzyl group, or $R^7$ and $R^8$ together with the adjacent nitrogen atom form a 1-pyrrolidinyl group) or $-OR^9$ (wherein $R^9$ is a hydrogen atom or a $C_{1-4}$ alkyl group); $R^4$ is a hydrogen atom; and n is 0 or 1.

The compounds of the formula (I) can, for example, be prepared by the following methods:

Method A:

0163240

This method comprises introducing a substituent of the formula: $-(CH_2)_nR^3$, wherein $R^3$ and n are as defined above, on the imidazole nucleus by allowing to react a compound of the formula:

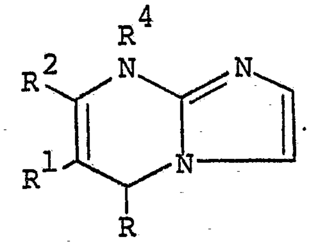

(II)

wherein R, $R^1$, $R^2$ and $R^4$ are as defined above, with an electrophilic reagent.

The reaction is performed in the presence of a suitable solvent.

The reaction includes an aminomethylation by Mannich reaction of formalin, paraformaldehyde or 1,3,5-trioxan with various amines; formylation by Vilsmeyer's reaction with Vilsmeyer's reagent; hydroxymethylation with formalin or paraformaldehyde in the presence of an acid or base catalyst; halogenation with a halogen or a halogenation reagent; acylation by Friedel-Crafts' reaction with an acyl halide in the presence of an acid catalyst; alkylation by Friedel-Crafts' reaction with an alkyl halide in the presence of an acid catalyst; or nitration with a nitrating reagent such as nitric acid.

Method B:

This method comprises allowing to react a compound of the formula:

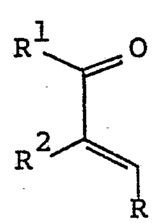

(III)

wherein R, $R^1$ and $R^2$ are as defined above, with a compound of the formula:

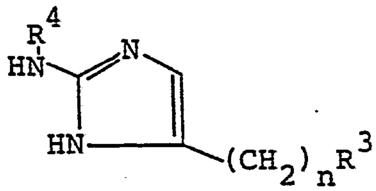

(IV)

wherein $R^3$, $R^4$ and n are as defined above, at room temperature or under heating, in the presence of a suitable solvent or without solvent.

Method C:

This method comprises allowing to react a compound of the formula:

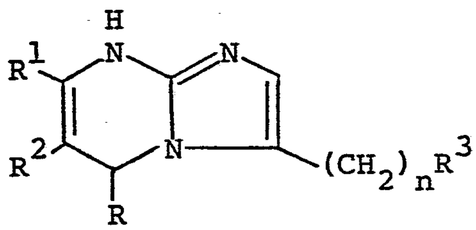

(V)

wherein R, $R^1$, $R^2$, $R^3$ and n are as defined above, with a compound of the formula:

$$(R_c)(R_d)N(CH_2)_m\text{-}X \qquad (VI)$$

wherein X is a reactive atom or group such as a halogen atom (e.g. chlorine or bromine) or a sulfonyloxy group (e.g. methanesulfonyloxy or toluenesulfonyloxy) and other symbols are as defined above, in the presence of a suitable solvent.

In this reaction, compounds introduced the substituent $R^4$ to the 1-position of imidazopyrimidine nucleus may be formed, but such side products can be removed by a conventional purification method such as fractional recrystallization or column chromatography.

Method D:

This method comprises converting a substituent or substituents of the compounds prepared according to the above Methods A, B and C into another or other substituents in accordance with a conventional synthetic manner.

Such manners include, for example, alkylation or acylation of an alcohol or an amine; halogenation; alkylsulfonylation or arylsulfonylation of an alcohol; amination, alkoxylation, alkylthiolation or C-C bond formation by substitution reaction with a nucleophilic reagent such as an amine, an alcohol, a mercaptan or a carbanion using a halogen, an amine, a quarternary ammonium salt or an ester as a leaving group; reduction of a nitro group, a cyano group or an amido group to an amine group; reduction of a ketone, an aldehyde or a carboxylic acid or its ester to an alcohol; hydrolysis of an ester to an alcohol or a carboxylic acid; decarboxylation of a carboxylic acid; amination or azido formation of a carboxylic acid or an activated carboxylic acid such as an acid halide, an acid anhydride or an ester; rearrangement of an acid amide or acid azide to an amine or a derivative of amine; and halogenation of an α-position of a ketone.

The solvents employed in Methods A, B and C indluce, for example, a lower alkanol (e.g. methanol, ethanol, propanol, isopropanol, butanol, tert-butanol or ethylene glycol), an aromatic hydrocarbon (e.g. benzene, toluene or xylene), an ether (e.g. diethyl ether, dimethoxyethane, diethylene glycol dimethyl ether, tetrahydrofuran or dioxane), a halogenated lower alkane (e.g. dichloromethane, chloroform or dichloroethane),

an acid amide (e.g. dimethylformamide, N-methylpyrrolidone or hexamethylphosphorotriamide), an ester (e.g. ethyl acetate or butyl acetate), a carboxylic acid (e.g. formic acid or acetic acid), a ketone (e.g. acetone or methyl ethyl ketone), nitromethane, acetonitrile, dimethylsulfoxide, sulforane or water, or a mixture thereof.

The compounds of formula (I) having an asymmetric carbon atom can be prepared as a racemate or an optically active isomer. The racemate can be devided into a desired optical isomer by means of a fractional recrystallization of a salt with an optically active acid. The compounds of formula (I) having at least two asymmetric carbon atoms can be prepared as an individual diastereomer or a mixture thereof. The individual diastereomer can be purified by means of fractional recrystallization or chromatography.

The compounds of formula (I), optical isomers thereof, diastereomers thereof or enantiomer thereof can be converted into pharmaceutically acceptable acid addition salts thereof with an inorganic acid such as hydrochloric, hydrobromic, nitric or phosphoric acids, an organic acid such as acetic, propionic, glycollic, succinic, maleic, fumaric, malic, tartric, citric, benzoic, cinnamic, mandelic, salicylic, 2-acetoxy-benzoic, nicotinic or isonicotinic acids, an organic sulfonic acid such as methanesulfonic, ethanesulfonic, 2-hydroxyethanesulfonic benzenesulfonic, p-toluenesulfonic or naphthalene-2-sulfonic acids, or ascorbic acid.

Since the compounds of the present invention exhibit calcium antagonistic, antihypertensive, cardiovasodilative,

cerebrovasodilative, cardiotonic, lipid peroxidation inhibiting and anticholinergic activity and low toxicity, and are characteristically weak in cardiac depressive activity as shown in the following pharmacological experiments, they are useful as drugs for the prophylaxis, treatment or ameriolation of coronary and cerebral circulatory diseases such as antihypertensive agetns, cardiotonics, vasoprophylactics, a drugs for the treatment of heart attack, heart failure or cardiac infarction, or drugs for the improvement of cerebral circulation.

The following pharmacological experiments explain the effects of the compounds according to the present invention in more detail. The test compounds employed are as below.

Compound A : Methyl 3-diethylaminomethyl-7-methyl-5-(3-nitrophenyl)-5,8-dihydroimidazo[1,2-a]pyrimidine-6-carboxylate hydrochloride

Compound B : Ethyl 3-diethylaminomethyl-7-methyl-5-(3-nitrophenyl)-5,8-dihydroimidazo[1,2-a]pyrimidine-6-carboxylate hydrochloride

Compound C : Ethyl 3-(N-benzyl-N-methylamino)methyl-7-methyl-5-(3-nitrophenyl)-5,8-dihydroimidazo[1,2-a]pyrimidine-6-carboxylate dihydrochloride monohydrate

Compound D : Ethyl 7-methyl-5-(3-nitrophenyl)-3-(1-pyrrolidinyl)-5,8-dihydroimidazo[1,2-a]pyrimidine-6-carboxylate

Compound E : Methyl 3-diethylaminomethyl-7-methyl-5-(2-trifluoromethylphenyl)-5,8-dihydroimidazo[1,2-a]pyrimidine-6-carboxylate

Compound F : Ethyl 3-diethylamino-7-methyl-5-(3-nitrophenyl)-5,8-dihydroimidazo[1,2-a]pyrimidine-6-carboxylate

0163240

Compound G : Ethyl 3-ethoxymethyl-7-methyl-5-(3-nitrophenyl)-5,8-dihydroimidazo[1,2-a]pyrimidine-6-carboxylate

Compound H : Methyl 3-dipropylaminomethyl-7-methyl-5-(3-nitrophenyl)-5,8-dihydroimidazo[1,2-a]pyrimidine-6-carboxylate

Compound I : Ethyl 3-diethylaminomethyl-7-methyl-5-(2-nitrophenyl)-5,8-dihydroimidazo[1,2-a]pyrimidine-6-carboxylate dihydrochloride monohydrate

Compound J : 2-Methoxyethyl 3-diethylaminomethyl-7-methyl-5-(3-nitrophenyl)-5,8-dihydroimidazo[1,2-a]pyrimidine-6-carboxylate

Compound K : Methyl 3-diisopropylaminomethyl-7-methyl-5-(3-nitrophenyl)-5,8-dihydroimidazo[1,2-a]pyrimidine-6-carboxylate

Compound L : Methyl 3-isopropoxymethyl-7-methyl-5-(3-nitrophenyl)-5,8-dihydroimidazo[1,2-a]pyrimidine-6-carboxylate

Compound M : Ethyl 7-methyl-3-nitro-5-(2-trifluoromethylphenyl)-5,8-dihydroimidazo[1,2-a]pyrimidine-6-carboxylate

Compound N : Ethyl 3-benzylaminomethyl-7-methyl-5-(3-nitrophenyl)-5,8-dihydroimidazo[1,2-a]pyrimidine-6-carboxylate dihydrochloride

Compound O : Ethyl 3-butylaminomethyl-7-methyl-5-(3-nitrophenyl)-5,8-dihydroimidazo[1,2-a]pyrimidine-6-carboxylate dihydrochloride

Compound P : Methyl 7-methyl-5-(3-nitrophenyl)-3-(1-pyrrolidinylmethyl)-5,8-dihydroimidazo[1,2-a]pyrimidine-6-carboxylate dihydrochloride

Compound Q : 2-Piperidino-1-phenylethyl 3-diethylaminomethyl-7-methyl-5-(3-nitrophenyl)-5,8-dihydroimidazo[1,2-a]pyrimidine-6-carboxylate (ß-form)

Compound R : Ethyl 7-methyl-5-(3-nitrophenyl)-3-(N-benzyl-N-methyl)amino-5,8-dihydroimidazo[1,2-a]pyrimidine-6-carboxylate

Compound S : Methyl 7-methyl-5-(3-nitrophenyl)-3-(2-trifluoromethylphenyl)aminomethyl-5,8-dihydroimidazo[1,2-a]pyrimidine-6-carboxylate

Compound T : Methyl 3-diethylaminomethyl-7-methyl-5-(2-nitrophenyl)-5,8-dihydroimidazo[1,2-a]pyrimidine-6-carboxylate

Pharmacological experiment 1: Calcium antagonistic activity in rabbit aorta

Rabbits of either sex, weighing 1.5 - 3.0 kg were killed by a blow on the head and thoracic aorta were isolated. Aorta were cut into ring segments of about 2 mm and were suspended in 40 ml chambers filled with Krebs-Henseleit solution bubbled with a gas mixture of 95% $O_2$ and 5% $CO_2$ (pH 7.4) and kept at 37°C. The contraction was isometrically recorded with a force displacement transducer. The 50% inhibitory concentration ($IC_{50}$, M) of test compounds on the contraction induced by 45 mM of potassium chloride (KCl) or $10^{-6}$ M of phenylephrine (PE, (-)-m-hydroxy-α-(methylaminomethyl)benzyl alcohol) were measured. The results are summerized in the following Table 1.

0163240

Table 1

| Test compound | $IC_{50}$ (M) | |
|---|---|---|
| | KCl contraction | PE contraction |
| A | $7.4 \times 10^{-8}$ | $>10^{-5}$ |
| B | $3.0 \times 10^{-8}$ | $>10^{-5}$ |
| E | $4.7 \times 10^{-7}$ | $>10^{-5}$ |
| F | $6.0 \times 10^{-7}$ | $>10^{-5}$ |
| G | $1.5 \times 10^{-7}$ | $>10^{-5}$ |
| I | $4.0 \times 10^{-7}$ | $>10^{-5}$ |
| J | $1.9 \times 10^{-7}$ | $>10^{-5}$ |
| P | $7.0 \times 10^{-7}$ | $>10^{-5}$ |

Table 1 shows that test compounds relaxed the contraction induced by KCl in a concentration-dependent manner, but that there was no effect on the contraction induced by PE.

Pharmacological experiment 2: Effect on coronary blood flow

Adult mongrel dogs were anesthetized with sodium pentobarbital (30 mg/kg, i.v.). According to the method of Yago et al described in Folia Pharmacol. Japon, vol. 57, 380 (1961), the left coronary artery was perfused and its blood flow was measured. Test compound was injected into the coronary artery at a volume of 10-30 μl. The effects of test compound on coronary blood flow were expressed as $ED_{50}$ (μg), a dose required to increase the coronary blood flow by a half of the effects of 3 μg of nifedipine. The half-life (T 1/2, minute) was measured as the duration of effects. The results are summarized in the Table 2.

0163240

Table 2

| Test Compound | $ED_{50}$ (μg) | T 1/2 (min.) |
|---|---|---|
| A | 2 | 1.0 |
| B | 3 | 1.5 |
| C | 10 | 1.5 |
| D | 8 | 1.3 |
| E | 5 | 1.0 |
| F | 12 | 1.0 |
| G | 7 | 1.0 |
| H | 7 | 1.0 |
| I | 10 | 2.0 |
| J | 5 | 1.7 |
| K | 6 | 1.5 |
| L | 5 | 1.0 |
| M | 5 | 1.0 |

Pharmacological experiment 3: Effect on vertebral blood flow

Adult mongrel dogs were anesthetized with sodium pentobarbital (25 mg/kg, i.v.). The right vertebral artery was perfused and the blood flow was measured. Test compound was injected in the vertebral artery. The percentage of maximum percentage increase of blood flow by the injection of 100 μg of papaverine hydrochloride (1-[(3,4-dimethoxyphenyl) methyl]-6,7-dimethoxyisoquinoline hydrochloride) in vertebral artery was taken as 100%. The effects of test compound on vertebral artery were expressed as $ED_{100}$ (μg), a dose required to obtain 100% of the percentage increase of blood flow. Half-life (T 1/2, minute) was measured as the duration of

0163240

effects. The results are summerized in the following Table 3.

Table 3

| Test Compound | $ED_{100}$ (μg) | T 1/2 (min.) |
|---|---|---|
| A | 2 | 0.9 |
| B | 10 | 0.8 |
| C | 5 | 1.5 |
| H | 2 | 1.0 |
| N | 10 | 0.7 |
| O | 15 | 0.7 |
| P | 6 | 0.6 |
| Q | 18 | 4.0 |

Pharmacological experiment 4: Effect on lipid peroxidation

Male Sprague-Dawley rats weighing 300-350 g were used. According to the method of Shimada et al described in Biochim. Biophys. Acta, vol. 572, 531 (1979), the liver mitochondria were isolated and ascorbic acid, ferrous sulfate, potassium chloride and Tris-HCl buffer were added. To the solution was added test compound dissolved in dimethyl sulfoxide, and the mixture was incubated at 25°C for 30 minutes. The amount of malondialdehyde was determined by the thiobarbituric acid method and the 50% inhibitory concentration ($IC_{50}$, μmol) of test compound on the formation of malondialdehyde was measured. The results are summerized in the following Table 4.

Table 4

| Test Compound | $IC_{50}$ (μmol) |
|---|---|
| A | 50 |
| B | 20 |
| D | 0.2 |
| E | 15 |
| F | 2 |
| G | 10 |
| R | 0.7 |

Pharmacological experiment 5: Effect on hypertension

Each of 30 mg/kg of test compounds A and Q was orally administered to five male spontaneously hypertensive rats and the blood pressure was measured by the tail cuff method. The blood pressure lowering value after 1 hour administration of test compound A was 48 mmHg and that value after 5 hours administration of test compound Q was 64 mmHg.

Pharmacological experiment 6: Anticholinergic activity

Trachea of guinea pig, weighing 380-550 g, was isolated and prepared its strip chain preparation. The preparation was mounted in organ bath filled with Krebs-Henseleit solution gassed with 95% $O_2$ and 5% $CO_2$. Length of the preparation was measured by isometric transducer (MEC, ME-4012). The preparation was contracted by $10^{-6}$ M of acetylcholine (ACh) and then, test compounds were added cumulatively into the bath to observe relaxant effect. Test compounds A, B and P ($10^{-7}$-$10^{-5}$ M) relaxed ACh-contracted preparation in a concentration-dependent manner. The 50% inhibitory

concentration ($IC_{50}$) of test compounds A, B and P on ACh-induced contraction were $1.6 \times 10^{-6}$, $4.0 \times 10^{-6}$ and $2.3 \times 10^{-6}$ M, respectively.

Since $IC_{50}$ of atropine was $7.4 \times 10^{-10}$ M, these compounds have moderate anticholinergic activity which were about 1/5000-1/2000 that of atropine.

Pharmacological experiment 7: Toxicity

Test compounds A, B, C, E and S were orally administered to five ddY strain mice. All mice were survived at the oral dose of 1000 mg/kg.

In view of the results above, the compounds of the present invention are proved to be useful as drugs for the treatment of coronary and cerebral circulatory diseases.

The compounds of the present invention can be administered orally or parenterally in the form of a pharmaceutical composition without harmful side effects to the patients. The pharmaceutical compositions comprise a therapeutically effective amount of the compounds of the present invention and a pharmaceutically acceptable additive such as an excipient, an extender, a diluent or a solubilizer, and can take the form of tablets, granules, powder, capsules, injectable solution, ointments or suppositories. The choice of additive is determined by the preferred form of administration, the solubility of the compound and standard pharmaceutical practice.

Formulation Example

The 20 or 50 mg tablets can be prepared according to the following compositions:

| | 20 mg Tablets | 50 mg Tablets |
|---|---|---|
| Compound A | 20.0 mg | 50.0 mg |
| Lactose | 64.5 | 67.0 |
| Corn starch | 20.0 | 25.0 |
| Crystalline cellulose | 10.0 | 20.0 |
| Methyl cellulose | 1.0 | 1.5 |
| Talc | 4.0 | 6.0 |
| Magnesium stearate | 0.5 | 0.5 |
| | 120.0 mg | 170.0 mg |

The daily dose of the compounds of the present invention for human adults usually ranges from 100 to 500 mg in a single or multiple dose, but it may vary depending upon the age, body weight, and/or severity of the condition to be treated as well as the response to the medication.

The present invention will be explained by the following preparative examples and examples in more detail, but these examples are not to be construed as limiting the present invention:

Preparative Example 1

To sodium ethoxide prepared from 1.2 g of sodium and 50 ml of ethanol are added 6.6 g of 2-aminoimidazole sulfate and 150 ml of ethanol, and the mixture is stirred at room temperature and filtered. To the filtrate is added 9.2 g of ethyl 2-acetyl-2-hexanoate and refluxed for 120 minutes. The ethanol is distilled off under reduced pressure and the resultant crystals are recrystallized from isopropanol to give ethyl 7-methyl-5-propyl-5,8-dihydroimidazo[1,2-a]pyrimidine-6-carboxylate, melting at 144-145°C.

0163240

Preparative Example 2

To sodium ethoxide prepared from 3.5 g of sodium and 100 ml of ethanol are added 20 g of 2-aminoimidazole sulfate and 200 ml of ethanol, and the mixture is stirred at room temperature and an insoluble material is filtered off. To the filtrate is added 39 g of ethyl α-acetyl-3-nitrocinnamate and stirred at 50°C for 3 hours. The precipitated yellow crystals are filtered to give 18 g of ethyl 7-methyl-5-(3-nitrophenyl)-5,8-dihydroimidazo[1,2-a]pyrimidine-6-carboxylate, melting at 242-243°C.

Preparative Example 3

To a solution of 4.5 g of 2-aminoimidazole sulfate and 1.3 g of sodium hydroxide in 11 ml of water is added 50 ml of ethanol, and the mixture is dried over anhydrous magnesium sulfate. To the resultant solution is added 10 g of ethyl 4,4-dimethoxy-2-(3-nitrobenzylidene)-3-oxo-butyrate, and the mixture is stirred at 50°C for 30 minutes and then refluxed under for 6 hours. After cooling, the precipitated yellow crystals are filtered and purified by column chromatography on silica gel with chloroform-methanol eluents and then recrystallized from methanol to give 6 g of ethyl 7-dimethoxymethyl-5-(3-nitrophenyl)-5,8-dihydroimidazo[1,2-a]-pyrimidine-6-carboxylate, melting at 190-191°C with decomposition.

Preparative Example 4

To a solution of 19 g of ethyl 7-methyl-5-(3-nitrophenyl)-5,8-dihydroimidazo[1,2-a]pyrimidine-6-carboxylate in 190 ml of acetic acid is added dropwise 9.3 g of bromine in 45 ml of acetic acid at room temperature for about 10 minutes. After

stirring at room temperature for 30 minutes, the acetic acid is distilled off under reduced pressure and the residue is extracted with chloroform. The extract is washed with water and dried, and then the solvent is distilled of under reduced pressure. The residue is purified by column chlomatography on silica gel to give 6 g of ethyl 3-bromo-7-methyl-5-(3-nitrophenyl)-5,8-dihydroimidazo[1,2-a]pyrimidine-6-carboxylate.

Preparative Example 5

To a crude 2-piperidino-1-phenylethyl α-acetyl-3-nitrocinnamate, which is prepared from 30 g of 3-nitrobenzaldehyde and 67 g of 2-piperidino-1-phenylethyl acetoacetate, is added a solution of 2-aminoimidazole in 500 ml of ethanol and the mixture is stirred at 50°C for 5 hours. The 2-aminoimidazole is prepared from 26.4 g of 2-aminoimidazole sulfate and 8 g of sodium hydroxide. After cooling, the precipitated crystals are filtered and recrystallized from ethanol to give 9.7 g of one diastereomer of 2-piperidino-1-phenylethyl 7-methyl-5-(3-nitrophenyl)-5,8-dihydroimidazo[1,2-a]pyrimidine-6-carboxylate, melting at 205-206°C.

The filtrate is concentrated under reduced pressure and the residue is purified by column chromarography on silica gel with chloroform-ethyl acetate-ethanol eluants. The resultant crystals are recrystallized from chloroform-ethanol to give 4.8 g of another diastereomer of 2-piperidino-1-phenylethyl 7-methyl-5-(3-nitrophenyl)-5,8-dihydroimidazo[1,2-a]pyrimidine-6-carboxylate, melting at 216-217°C with decomposition.

Example 1

A mixture of 0.83 g of paraformaldehyde, 2.2 g of diethylamine and 35 ml of acetic acid is stirred at room temperature, and to the resultant mixture is added 4.6 g of ethyl 7-methyl-5-propyl-5,8-dihydroimidazo[1,2-a]pyrimidine-6-carboxylate and stirred at 50°C for 5.5 hours. The acetic acid is distilled off under reduced pressure, and then the residue is extracted with chloroform and the extract is dried. After the chloroform is distilled off under reduced pressure, the residue is converted into hydrochloride and recrystallized from ethanol to give 3.4 g of ethyl 3-diethylaminomethyl-7-methyl-5-propyl-5,8-dihydro[1,2-a]pyrimidine-6-carboxylate as white crystals, melting at 150-152°C with decomposition.

Example 2

A mixture of 1.1 g of paraformaldehyde, 3.3 g of diethylamine and 80 ml of acetic acid is stirred, and to the whole mixture is added 10 g of ethyl 7-methyl-5-(3-nitrophenyl)-5,8-dihydroimidazo[1,2-a]pyrimidine-6-carboxylate and stirred at 70°C for 3 hours. The resultant mixture is poured into aqueous potassium carbonate to neutralize and extracted with ethyl acetate. The extract is dried and the solvent is distilled off under reduced pressure. The residue is recrystallized from ethanol to give 9.0 g of ethyl 3-diethylaminomethyl-7-methyl-5-(3-nitrophenyl)-5,8-dihydroimidazo [1,2-a]pyrimidine-6-carboxylate as yellow crystals, melting at 183-185°C.

6 g of the above product is converted into the coresponding hydrochloride with ethanolic hydrochloric acid and recrystallized

from ethanol to give 4.2 g of ethyl 3-diethylaminomethyl-7-methyl-5-(3-nitrophenyl)- 5,8-dihydroimidazo[1,2-a]pyrimidine-6-carboxylate hydrochloride as pale yellow crystals, melting at 164-165°C.

Example 3

To a mixture of 0.6 g of paraformaldehyde, 2.1 g of benzylamine and 30 ml of acetic acid is added 3.3 g of ethyl 7-methyl-5-(3-nitrophenyl)-5,8-dihydroimidazo[1,2-a]pyrimidine-6-carboxylate and the whole mixture is stirred at 60°C for 5 hours. The resultant mixutre is neutralized with aqueous potassium carbonate and extracted with chloroform. After the extract is dried, the solvent is concentrated under reduced pressure. The resultant crystals are subjected to column chromatography on 80 g of silica gel with chloroform-ethanol eluants and recrystallized from ethanol to give 2.2 g of ethyl 3-benzylaminomethyl-7-methyl-5-(3-nitrophenyl)-5,8-dihydroimidazo[1,2-a]pyrimidine-6-carboxylate, melting at 173-175°C.

2.2 g of the above product is treated with ethanolic hydrochloric acid to convert into the coresponding hydrochloride and recrystallized from ethanol to give 1.1 g of ethyl 3-benzylaminomethyl-7-methyl-5-(3-nitrophenyl)-5,8-dihydroimidazo[1,2-a]pyrimidine-6-carboxylate dihydrochloride as white crystals, melting at 216-217°C.

Example 4

To a mixture of 1.7 g of paraformaldehyde, 4,5 g of diethylamine and 150 ml of acetic acid is added 15 g of ethyl 7-dimethoxymethyl-5-(3-nitrophenyl)-5,8-dihydroimidazo[1,2-a]

pyrimidine-6-carboxylate, and the whole mixture is stirred at 50°C for 4 hours. After the acetic acid is distilled off under reduced pressure, aqueous sodium hydrogencarbonate is added to the residue to neutralize and the solution is extracted with chloroform. The extract is dried and concentrated under reduced pressure. The residue is purified by column chromatography on silica gel and recrystallized from a mixture of chloroform and ethanol to give 7.5 g of ethyl 3-diethylamino-methyl-7-dimethoxymethyl-5-(3-nitrophenyl)-5,8-dihydroimidazo-[1,2-a]pyrimidine-6-carboxylate as yellow crystals, melting at 198-199°C.

Example 5

To a solution of 3 g of ethyl 3-bromo-7-methyl-5-(3-nitrophenyl)-5,8-dihydroimidazo[1,2-a]pyrimidine-6-carboxylate in 30 ml of dimethylformamide is added 3.2 g of diethylamine, and the mixture is stirred at 80°C for 5 hours. After the solvent is distilled off under reduced pressure and the residue is extracted with chloroform, the extract is washed with water and dried. The chloroform is distilled off under reduced pressure, and then the residue is crystallized from isopropyl ether and recrystallized from ethanol to give 0.7 g of ethyl 3-diethyl-amino-7-methyl-5-(3-nitrophenyl)-5,8-dihydroimidazo [1,2-a]pyrimidine-6-carboxylate as yellow crystals, melting at 204-206°C.

Example 6

To a mixture of 0.5 g of paraformaldehyde, 1.6 g of diethylamine and 25 ml of acetic acid is added 5.4 g of one diastereomer of 2-piperidino-1-phenylethyl 7-methyl-5-

(3-nitrophenyl)-5,8-dihydroimidazo[1,2-a]pyrimidine-6-carboxylate, and the whole mixture is stirred at 70°C for 3 hours. After the acetic acid is distilled off, the residue is neutralized with aqueous sodium hydrogencarbonate and then is extracted with chloroform. The chloroform layer is whshed with water, dried and concentrated. The residue is purified by column chromatography on silica gel with chloroform-methanol eluants and recrystallized from a mixture of chloroform and ethanol to give 3.5 g of a diastereomer of 2-piperidino-1-phenyl-ethyl 3-diethylaminomethyl-7-methyl-5-(3-nitrophenyl)-5,8-dihydroimidazo[1,2-a]-pyrimidine-6-carboxylate as pale yellow crystals, melting at 197-198°C ( α-form).

1.5 g of another diastereomer (β-form) of 2-piperidino-1-phenylethyl 3-diethylamino-methyl-7-methyl-5-(3-nitrophenyl)-5,8-dihydroimidazo[1,2-a]pyrimidine-6-carboxylate as pale yellow crystals, melting at 203-205°C with decomposition can be prepared from 4.9 g of another diastereomer of 2-piperidino-1-phenylethyl 7-methyl-5-(3-nitrophenyl)-5,8-dihydroimidazo[1,2-a]pyrimidine-6-carboxylate in a similar manner.

Example 7

To a solution of 3.0 g of methyl 3-diethylaminomethyl-7-methyl-5-(3-nitrophenyl)-5,8-dihydroimidazo[1,2-a]pyrimidine-6-carboxylate in dimethylformamide is added 0.3 g of sodium hydride under ice-cooling. The mixture is stirred under ice-cooling for 30 minutes, at 30°C for an hour and at 50°C for 4 hours. The reaction mixture is extracted with ethyl acetate, and the extract is washed with water and dried over magnesium

sulfate. The ethyl acetate was distilled off and the residue is treated with a hydrochloric acid-isopropanol solution. The resultant hydrochloride is recrystallized from methanol to give methyl 3-diethylaminomethyl-7-methyl-8-(2-morpholinoethyl)-5-(3-nitrophenyl)-5,8-dihydroimidazo[1,2-a]pyrimidine-6-carboxylate, melting at 162-163°C with decomposition.

The following compounds can be prepared according to any methods of above examples:

(8) Ethyl 3-(N-benzyl-N-methylamino)methyl-7-methyl-5-(3-nitrophenyl)-5,8-dihydroimidazo[1,2-a]pyrimidine-6-carboxylate dihydrochloride monohydrate, melting at 179-181°C with decomposition

(9) Methyl 3-diethylaminomethyl-7-methyl-5-(3-nitrophenyl)-5,8-dihydroimidazo[1,2-a]pyrimidine-6-carboxylate hydrochloride, melting at 172-173°C with decomposition

(10) Ethyl 3-isopropylaminomethyl-7-methyl-5-(3-nitrophenyl)-5,8-dihydroimidazo[1,2-a]pyrimidine-6-carboxylate, melting at 210-211°C

(11) Methyl 3-diethylaminomethyl-7-methyl-5-(2-thienyl)-5,8-dihydroimidazo[1,2-a]pyrimidine-6-carboxylate hydrochloride, melting at 170-171°C with decomposition

(12) Ethyl 3-diethylaminomethyl-7-methyl-5-phenyl-5,8-dihydroimidazo[1,2-a]pyrimidine-6-carboxylate dihydrochloride dihydrate, melting at 181-182°C with decomposition

(13) Methyl 5-(2-chlorophenyl)-3-diethylaminomethyl-7-methyl-5,8-dihydroimidazo[1,2-a]pyrimidine-6-carboxylate hydrochloride 1/2 hydrate, melting at 167-169°C with decomposition

(14) Ethyl 7-methyl-5-(3-nitrophenyl)-3-(1-piperadinyl)methyl-5,8-dihydroimidazo[1,2-a]pyrimidine-6-carboxylate trihydrochloride monohydrate, melting at above 300°C

(15) Ethyl 3-diethylaminomethyl-7-methyl-5-(2-methylphenyl)-5,8-dihydroimidazo[1,2-a]pyrimidine-6-carboxylate dihydrochloride 1/2 hydrate, melting at 183-186°C with decomposition

(16) Ethyl 3-butylaminomethyl-7-methyl-5-(3-nitrophenyl)-5,8-dihydroimidazo[1,2-a]pyrimidine-6-carboxylate dihydrochloride, melting at 211-212°C with decomposition

(17) Ethyl 3-diethylaminomethyl-7-methyl-5-(2-nitrophenyl)-5,8-dihydroimidazo[1,2-a]pyrimidine-6-carboxylate dihydrochloride monohydrate, melting at 183-185°C with decomposition

(18) Ethyl 3-diethylaminomethyl-7-methyl-5-(3-trifluoromethylphenyl)-5,8-dihydroimidazo[1,2-a]pyrimidine-6-carboxyalte dihydrochloride, melting at 180-181°C with decomposition

(19) Ethyl 3-(1-imidazolyl)methyl-7-methyl-5-(3-nitrophenyl)-5,8-dihydroimidazo[1,2-a]pyrimidine-6-carboxylate hydrochloride monohydrate, melting at 175-177°C with decomposition

(20) Ethyl 3-diethylaminomethyl-7-methyl-5-(2-trifluoromethylphenyl)-5,8-dihydroimidazo[1,2-a]pyrimidine-6-carboxylate hydrochloride 1/3 hydrate, melting at 130-133°C with decomposition

(21) Methyl 7-methyl-3-dimethylaminomethyl-5-(3-nitrophenyl)-5,8-dihydroimidazo[1,2-a]pyrimidine-6-carboxylate dihydrochloride, melting at 215°C with decomposition

(22) Methyl 3-(4-(2-furylcarbonyl)-1-piperadinyl)methyl-7-methyl-5-(3-nitrophenyl)-5,8-dihydroimidazo[1,2-a]pyrimidine-6-carboxylate dihydrochloride 1/2 hydrate, melting at 188-190°C with decomposition

0163240

(23) Methyl 7-methyl-5-(3-nitrophenyl)-3-(1-pyrrolidinylmethyl)-5,8-dihydroimidazo[1,2-a]pyrimidine-6-carboxylate dihydrochloride, melting at 208-210°C with decomposition

(24) Methyl 3-dihexylaminomethyl-7-methyl-5-(3-nitrophenyl)-5,8-dihydroimidazo[1,2-a]pyrimidine-6-carboxylate, melting at 165°C

(25) Methyl 7-methyl-5-(3-nitrophenyl)-3-piperidinomethyl-5,8-dihydroimidazo[1,2-a]pyrimidine-6-carboxylate hydrochloride 3/2 hydrate, melting at 180-181°C with decomposition

(26) Methyl 7-methyl-3-morpholinomethyl-5-(3-nitrophenyl)-5,8-dihydroimidazo[1,2-a]pyrimidine-6-carboxylate dihydrochloride 3/2 hydrate, melting at 174-175°C with decomposition

(27) Ethyl 3-diethylaminomethyl-5,7-dimethyl-5,8-dihydroimidazo-[1,2-a]pyrimidine-6-carboxylate hydrochloride, melting at 185-186°C with decomposition

(28) Ethyl 3-diethylaminomethyl-5-(2-methoxyphenyl)-7-methyl-5,8-dihydroimidazo[1,2-a]pyrimidine-6-carboxylate hydrochloride 1/3 hydrate, melting at 150-152°C with decomposition

(29) Methyl 3-ethylaminomethyl-7-methyl-5-(3-nitrophenyl)-5,8-dihydroimidazo[1,2-a]pyrimidine-6-carboxylate dihydrochloride 1/3 hydrate, melting at 218-219°C with decomposition

(30) Methyl 5-(2,1,3-benzoxadiazole-4-yl)-3-diethylaminomethyl-7-methyl-5,8-dihydroimidazo[1,2-a]pyrimidine-6-carboxylate dihydrochloride 1/2 hydrate, melting at 158-160°C

(31) Methyl 3-diethylaminomethyl-7-methyl-5-(4-nitrophenyl)-5,8-dihydroimidazo[1,2-a]pyrimidine-6-carboxylate hydrochloride, melting at 164-166°C with decomposition

(32) Methyl 3-diethylaminomethyl-7-methyl-5-(2-trifluoromethylphenyl)-5,8-dihydroimidazo[1,2-a]pyrimidine-6-carboxylate, melting at 195-196°C with decomposition

(33) Methyl 3-aminomethyl-7-methyl-5-(3-nitrophenyl)-5,8-dihydroimidazo[1,2-a]pyrimidine-6-carboxylate dihydrochloride, melting at above 340°C

(34) Methyl 7-methyl-5-(3-nitrophenyl)-3-(2-trifluoromethylphenyl)aminomethyl-5,8-dihydroimidazo[1,2-a]pyrimidine-6-carboxylate, melting at 219-220°C

(35) Methyl 5-(2-cyanophenyl)-3-diethylaminomethyl-7-methyl-5,8-dihydroimidazo[1,2-a]pyrimidine-6-carboxylate hydrochloride, melting at 189°C with decomposition

(36) Ethyl 3-N-butyl-N-butyrylaminomethyl-5-(3-nitrophenyl)-7-methyl-5,8-dihydroimidazo[1,2-a]pyrimidine-6-carboxylate, melting at 192-193°C.

(37) Ethyl 3-(4-butyryl-1-piperazinyl)methyl-7-methyl-5-(3-nitrophenyl)-5,8-dihydroimidazo[1,2-a]pyrimidine-6-carboxylate hydrochloride, melting at 154-156°C

(38) Methyl 3-diethylaminomethyl-7-methyl-5-(3-nitrophenyl)-5,8-dihydroimidazo[1,2-a]pyrimidine-6-carboxylate, melting at 205°C

(39) Methyl 3-diethylaminomethyl-7-methyl-5-(2-nitrophenyl)-5,8-dihydroimidazo[1,2-a]pyrimidine-6-carboxylate, melting at 197-198°C with decomposition

(40) Ethyl 3-diethylaminomethyl-7-hydroxymethyl-5-(3-nitrophenyl)-5,8-dihydroimidazo[1,2-a]pyrimidine-6-carboxylate, melting at 179-180°C with decomposition

(41) 2-Piperidinoethyl 3-diethylaminomethyl-7-methyl-5-(2-trifluoromethylphenyl)-5,8-dihydroimidazo[1,2-a]pyrimidine-6-carboxylate, melting at 142-143°C

(42) Ethyl 7-acetoxymethyl-3-diethylaminomethyl-5-(3-nitrophenyl)-5,8-dihydroimidazo[1,2-a]pyrimidine-6-carboxylate, melting at 148-149°C

(43) Ethyl 3-diethylaminomethyl-7-hydroxyiminomethyl-5-(3-nitrophenyl)-5,8-dihydroimidazo[1,2-a]pyrimidine-6-carboxylate, melting at 200-201°C with decomposition

(44) 2-Methoxyethyl 3-diethylaminomethyl-7-methyl-5-(3-nitrophenyl)-5,8-dihydroimidazo[1,2-a]pyrimidine-6-carboxylate, melting at 165°C

(45) Ethyl 3-ethoxymethyl-7-methyl-5-(3-nitrophenyl)-5,8-dihydroimidazo[1,2-a]pyrimidine-6-carboxylate, melting at 183-186°C

(46) Ethyl 3-diethylaminomethyl-7-methyl-5,8-dihydroimidazo-[1,2-a]pyrimidine-6-carboxylate, melting at 218-219°C with decomposition

(47) Methyl 3-dipropylaminomethyl-7-methyl-5-(3-nitrophenyl)-5,8-dihydroimidazo[1,2-a]pyrimidine-6-carboxylate, melting at 188-189°C

(48) Methyl 3-diisopropylaminomethyl-7-methyl-5-(3-nitrophenyl)-5,8-dihydroimidazo[1,2-a]pyrimidine-6-carobxylate, melting at 214-215°C

(49) Methyl 3,7-dimethyl-5-(3-nitrophenyl)-5,8-dihydroimidazo-[1,2-a]pyrimidine-6-carboxylate, melting at 254-255°C

(50) Methyl 3-ethylthiomethyl-7-methyl-5-(3-nitrophenyl)-5,8-dihydroimidazo[1,2-a]pyrimidine-6-carboxylate, melting at 215°C

(51) Ethyl 3-diethylaminomethyl-7-methylcarbamoyloxymethyl-5-(3-nitrophenyl)-5,8-dihydroimidazo[1,2-a]pyrimidine-6-carboxylate, melting at 184-185°C with decomposition

(52) Ethyl 3-hydroxymethyl-7-methyl-5-(3-nitrophenyl)-5,8-dihydroimidazo[1,2-a]pyrimidine-6-carboxylate, melting at 228-229°C with decomposition

(53) 2-Diethylaminoethyl 3-dipropylaminomethyl-7-methyl-5-(3-nitrophenyl)-5,8-dihydroimidazo[1,2-a]pyrimidine-6-carboxylate, melting at 156-158°C

(54) 6-Cyano-3-diethylaminomethyl-7-methyl-5-(3-nitrophenyl)-5,8-dihydroimidazo[1,2-a]pyrimidine, melting at 195-196°C with decomposition

(55) Ethyl 7-methyl-3-(2-trifluoromethylphenyl)aminomethyl-5,8-dihydroimidazo[1,2-a]pyrimidine-6-carboxylate, melting at 185-187°C

(56) Methyl 3-isopropoxymethyl-7-methyl-5-(3-nitrophenyl)-5,8-dihydroimidazo[1,2-a]pyrimidine-6-carboxylate, melting at 201°C

(57) Methyl 3-benzyloxymethyl-7-methyl-5-(3-nitrophenyl)-5,8-dihydroimidazo[1,2-a]pyrimidine-6-carboxylate, melting at 206-207°C

(58) 2-Methoxy-1-phenylethyl 3-diethylaminomethyl-7-methyl-5-(3-nitrophenyl)-5,8-dihydroimidazo[1,2-a]pyrimidine-6-carboxylate, melting point of α-form: 193°C; melting point of β-form: 191°C

(59) Ethyl 7-methyl-5-(3-nitrophenyl)-3-dipropylamino-5,8-dihydroimidazo[1,2-a]pyrimidine-6-carboxylate, melting at 205-206°C

(60) Ethyl 7-methyl-5-(3-nitrophenyl)-3-(N-benzyl-N-methylamino)-5,8-dihydroimidazo[1,2-a]pyrimidine-6-carboxylate,

melting at 182-183°C

(61) Ethyl 7-methyl-5-(3-nitrophenyl)-3-(1-pyrrolidinyl)-5,8-dihydroimidazo[1,2-a]pyrimidine-6-carboxylate, melting at 207-208°C

(62) Ethyl 7-methyl-3-nitro-5-(2-trifluoromethylphenyl)-5,8-dihydroimidazo[1,2-a]pyrimidine-6-carboxylate, melting at 185-186°C

(63) Ethyl 3-formyl-7-methyl-5-(3-nitrophenyl)-5,8-dihydroimidazo[1,2-a]pyrimidine-6-carboxylate, melting at 229-231°C

(64) Ethyl 3-acetoxymethyl-7-methyl-5-(3-nitrophenyl)-5,8-dihydroimidazo[1,2-a]pyrimidine-6-carboxylate, melting at 220°C with decomposition

(65) 6-Methoxycarbonyl-7-methyl-5-(3-nitrophenyl)-5,8-dihydroimidazo[1,2-a]pyrimidine-3-carboxylic acid, melting at 253-254°C

(66) 6-Ethoxycarbonyl-7-methyl-5-(3-nitrophenyl)-5,8-dihydroimidazo[1,2-a]pyrimidine-3-propionic acid, melting at 205°C with decomposition

(67) Ethyl 3-(3-hydroxypropyl)-7-methyl-5-(3-nitrophenyl)-5,8-dihydroimidazo[1,2-a]pyrimidine-6-carboxylate, melting at 183-184°C

(68) Ethyl 3-(2-diethylaminoethyl)-7-methyl-5-(3-nitrophenyl)-5,8-dihydroimidazo[1,2-a]pyrimidine-6-carboxylate

(69) Ethyl 3-(2-aminoethyl)-7-methyl-5-(3-nitrophenyl)-5,8-dihydroimidazo[1,2-a]pyrimidine-6-carboxylate

(70) Ethyl 7-methyl-3-(2-nitroethyl)-5-(3-nitrophenyl)-5,8-dihydroimidazo[1,2-a]pyrimidine-6-carboxylate, melting at 219 - 221°C.

(71) 2-Diethylaminoethyl 3-diisopropylaminomethyl-7-methyl-5-(3-nitrophenyl)-5,8-dihydroimidazo[1,2-a]pyrimidine-6-carboxylate

(72) Methyl 3-diisopropylaminomethyl-7-methyl-5-(2-pyridyl)-5,8-dihydroimidazo[1,2-a]pyrimidine-6-carboxylate

(73) 6-Cyano-3-dibutylaminomethyl-7-methyl-5-(3-nitrophenyl)-5,8-dihydroimidazo[1,2-a]pyrimidine

(74) Methyl 3-dibutylaminomethyl-7-methyl-5-(3-nitrophenyl)-5,8-dihydroimidazo[1,2-a]pyrimidine-6-carboxylate

(75) Methyl 3-N-[2-(3,4-dimethoxyphenyl)ethyl]-N-methylaminomethyl-7-methyl-5-(3-nitrophenyl)-5,8-dihydroimidazo[1,2-a]-pyrimidine-6-carboxylate

(76) Methyl 3-diethylaminomethyl-5-(2-furyl)-7-methyl-5,8-dihydroimidazo[1,2-a]pyrimidine-6-carboxylate

(77) Methyl 3-diethylaminomethyl-5-(3-nitrophenyl)-7-propyl-5,8-dihydroimidazo[1,2-a]pyrimidine-6-carboxylate

(78) Methyl 3-(2-diethylaminoethyl)-7-methyl-5-(3-nitrophenyl)-5,8-dihydroimidazo[1,2-a]pyrimidine-6-carboxylate

(79) Methyl 3-(3-diethylaminopropyl)-7-methyl-5-(3-nitrophenyl)-5,8-dihydroimidazo[1,2-a]pyrimidine-6-carboxylate

(80) Methyl 3-hydroxymethyl-7-methyl-5-(3-nitrophenyl)-5,8-dihydroimidazo[1,2-a]pyrimidine-6-carboxylate

(81) 1-Phenyl-2-(N-benzyl-N-methylamino)ethyl 3-diethylaminomethyl-7-methyl-5-(3-nitrophenyl)-5,8-dihydroimidazo[1,2-a]-pyrimidine-6-carboxylate

What is claimed is:

1. A dihydroimidazo[1,2-a]pyrimidine derivative of the formula:

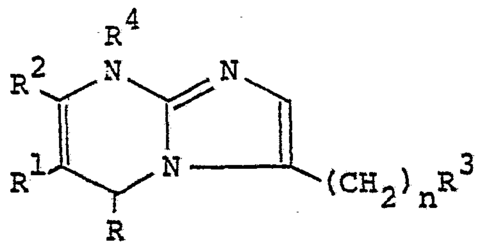

inclusive of an optical isomer thereof, a diastereomer thereof, and compounds selected from the group consisting of pharmaceutically acceptable acid addition salt forms thereof, hydrate forms thereof and mixtures thereof, wherein R is a hydrogen atom, a $C_{1-5}$ alkyl group, a heteroaryl group, a 2,1,3-benzoxadiazolyl group or a phenyl group which may be optionally substituted by a substituent selected from the group consisting of a halogen atom, a $C_{1-4}$ alkyl group, a trifluoromethyl group, a $C_{1-4}$ alkoxy group, a nitro group and a cyano group;

$R^1$ is a cyano group, a $C_{1-4}$ alkoxy-carbonyl group or a group represented by the formula of $-COOCH(R^5)CH_2R^6$ [wherein $R^5$ is a hydrogen atom or an aryl group and $R^6$ is a $C_{1-4}$ alkoxy group or a group represented by the formula of -N(Ra)(Rb) (wherein each of Ra and Rb is a $C_{1-4}$ alkyl group or an aralkyl group, or Ra and Rb together with the adjacent nitrogen atom form a heterocycle)];

$R^2$ is a $C_{1-4}$ alkyl group, a hydroxymethyl group, an acetoxymethyl group, a di-$C_{1-4}$ alkoxy-methyl group, a $C_{1-4}$ alkylcarbamoyloxymethyl group or a hydroxyiminomethyl group;

0163240

$R^3$ is a $C_{1-4}$ alkyl group, a formyl group, a carboxyl group, a nitro group or a group represented by the formula of $-N(R^7)(R^8)$ (wherein each of $R^7$ and $R^8$ is a hydrogen atom, a $C_{1-8}$ alkyl group, an aryl group, an aralkyl group or an acyl group, or $R^7$ and $R^8$ together with the adjacent nitrogen atom form a heterocycle), $-OR^9$ or $-SR^{10}$ (wherein each of $R^9$ and $R^{10}$ is a hydrogen atom, a $C_{1-8}$ alkyl group, an aralkyl group or an acyl group);

$R^4$ is a hydrogen atom or a group represented by the formula of $-(CH_2)_mN(Rc)(Rd)$ (wherein each of Rc and Rd is a $C_{1-4}$ alkyl group or an aralkyl group, or Rc and Rd together with the adjacent nitrogen aton form a heterocycle and m is an integer of from 1 to 3); and

n is 0 or an integer of from 1 to 3.

2. The compound of claim 1, wherein R is a hydrogen atom, a $C_{1-5}$ alkyl group, a thienyl group, a 2,1,3-benzoxadiazolyl group or a phenyl group which may be optionally substituted by a substituent selected from the group consisting of a halogen atom, a $C_{1-4}$ alkyl group, a trifluoromethyl group, a $C_{1-4}$ alkoxy group, a nitro group and a cyano group; $R^1$ is a cyano group, a $C_{1-4}$ alkoxy-carbonyl group or a group represented by the formula of $-COOCH(R^5)CH_2R^6$ [wherein $R^5$ is a hydrogen atom or a phenyl group and $R^6$ is a $C_{1-4}$ alkoxy group or a group represented by the formula of -N(Ra)(Rb) (wherein each of Ra and Rb is a $C_{1-4}$ alkyl group, or Ra and Rb together with the adjacent nitrogen atom form a piperidino group)]; $R^2$ is a $C_{1-4}$ alkyl group, a hydroxymethyl group, an acetoxymethyl group, a di-$C_{1-4}$ alkoxy-methyl group, a $C_{1-4}$ alkyl-carbamoyl-

oxymethyl group or a hydroxyiminomethyl group; $R^3$ is a $C_{1-4}$ alkyl group, a formyl group, a carboxyl group, a nitro group or a group represented by the formula of $-N(R^7)(R^8)$ (wherein each of $R^7$ and $R^8$ is a hydrogen atom, a $C_{1-8}$ alkyl group, a trifluoromethylphenyl group, a benzyl group or an acyl group, or $R^7$ and $R^8$ together with the adjacent nitrogen atom form a heterocycle selected from the group consisting of 1-pyrrolidinyl, piperidino, morpholino, 1-piperazinyl, 4-butyryl-1-piperazinyl, 4-(2-furylcarbonyl)-1-piperazinyl and imidazolyl groups, $-OR^9$ (wherein $R^9$ is a hydrogen atom, a $C_{1-4}$ alkyl group, a benzyl group or an acyl group) or $-SR^{10}$ (wherein $R^{10}$ is a $C_{1-4}$ alkyl group); $R^4$ is a hydrogen atom or a 2-morpholinoethyl group; and n is 0 or an integer of from 1 to 3.

3. The compound of claim 1, wherein R is a phenyl group substituted by a nitro group or a trifluoromethyl group; $R^1$ is a $C_{1-4}$ alkoxy-carbonyl group or a group represented by the formula of $-COOCH(R^5)CH_2R^6$ [wherein $R^5$ is a hydrogen atom or a phenyl group and $R^6$ is a methoxy group or a group represented by the formula of -N(Ra)(Rb) (wherein Ra and Rb together with the adjacent nitrogen atom form a piperidino group)]; $R^2$ is a $C_{1-4}$ alkyl group; $R^3$ is a group represented by the formula of $-N(R^7)(R^8)$ (wherein each of $R^7$ and $R^8$ is a $C_{1-4}$ alkyl group or a benzyl group, or $R^7$ and $R^8$ together with the adjacent nitrogen atom form a 1-pyrrolidinyl group) or $-OR^9$ (wherein $R^9$ is a hydrogen atom or a $C_{1-4}$ alkyl group); $R^4$ is a hydrogen atom; and n is 0 or 1.

4. The compound of claim 1:
Methyl 3-diethylaminomethyl-7-methyl-5-(3-nitrophenyl)-5,8-dihydroimidazo[1,2-a]pyrimidine-6-carboxylate or a pharmaceutically acceptable acid addition salt thereof.

5. The compound of claim 1:
Ethyl 3-diethylaminomethyl-7-methyl-5-(3-nitrophenyl)-5,8-dihydroimidazo[1,2-a]pyrimidine-6-carboxylate or a pharmaceutically acceptable acid addition salt thereof.

6. The compound of claim 1:
Methyl 3-diethylaminomethyl-7-methyl-5-(2-trifluoromethylphenyl)-5,8-dihydroimidazo[1,2-a]pyrimidine-6-carboxylate or a pharmaceutically acceptable acid addition salt thereof.

7. The compound of claim 1:
Methyl 3-dipropylaminomethyl-7-methyl-5-(3-nitrophenyl)-5,8-dihydroimidazo[1,2-a]pyrimidine-6-carboxylate or a pharmaceutically acceptable acid addition salt thereof.

8. The compound of claim 1:
Ethyl 3-(N-benzyl-N-methylamino)methyl-7-methyl-5-(3-nitrophenyl)-5,8-dihydroimidazo[1,2-a]pyrimidine-6-carboxylate or a pharmaceutically acceptable acid addition salt thereof and/or hydrate thereof.

9. The compound of claim 1:
Ethyl 7-methyl-5-(3-nitrophenyl)-3-(1-pyrrolidinyl)-5,8-dihydroimidazo[1,2-a]pyrimidine-6-carboxylate or a pharmaceutically acceptable acid addition salt thereof.

10. The compound of claim 1:
Methyl 3-isopropoxymethyl-7-methyl-5-(3-nitrophenyl)-5,8-dihydroimidazo[1,2-a]pyrimidine-6-carboxylate or a pharmaceutically acceptable acid addition salt thereof.

11. The compound of claim 1:

2-Piperidino-1-phenylethyl 3-diethylaminomethyl-7-methyl-5-(3-nitrophenyl)-5,8-dihydroimidazo[1,2-a]pyrimidine-6-carboxylate, a diastereomer thereof or a pharmaceutically acceptable acid addition salt thereof.

12. The compound of claim 1:

Ethyl 3-diethylamino-7-methyl-5-(3-nitrophenyl)-5,8-dihydroimidazo[1,2-a]pyrimidine-6-carboxylate or a pharmaceutically acceptable acid addition salt thereof.

13. The compound of claim 1:

Methyl 3-diisopropylaminomethyl-7-methyl-5-(3-nitrophenyl)-5,8-dihydroimidazo[1,2-a]pyrimidine-6-carboxylate or a pharmaceutically acceptable acid addition salt thereof.

14. The compound of claim 1:

2-Methoxyethyl 3-diethylaminomethyl-7-methyl-5-(3-nitrophenyl)-5,8-dihydroimidazo[1,2-a]pyrimidine-6-carboxylate or a pharmaceutically acceptable acid addition salt thereof.

15. The compound of claim 1:

Ethyl 7-methyl-3-nitro-5-(2-trifluoromethylphenyl)-5,8-dihydroimidazo[1,2-a]pyrimidine-6-carboxylate or a pharmaceutically acceptable acid addition salt thereof.

16. A method for preparing a dihydroimidazo[1,2-a]pyrimidine derivative of the formula:

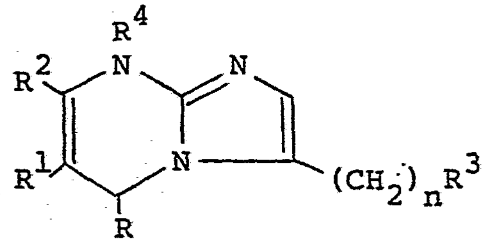

inclusive of an optical isomer thereof, a diastereomer thereof, and compounds selected from the group consisting of pharmaceutically acceptable acid addition salt forms thereof, hydrate forms thereof and mixtures thereof, which comprises:

(1) allowing to react a compound of the formula:

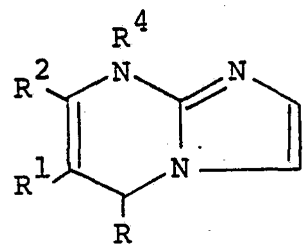

with an electrophilic reagent,

(2) allowing to react a compound of the formula:

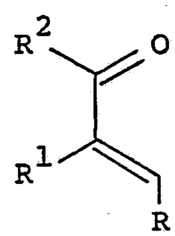

with a compound of the formula:

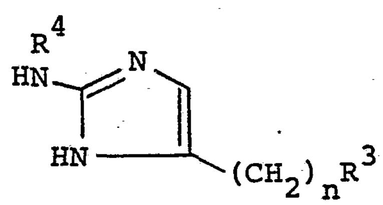

(3) allowing to react a compound of the formula:

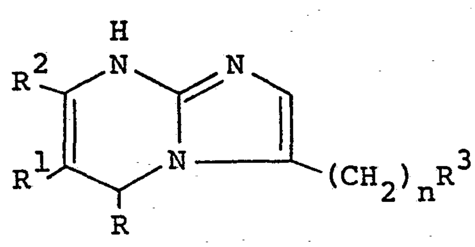

with a compound of the formula:

$$(Rc)(Rd)N(CH_2)_m—X$$

or if desired, (4) converting the compound obtained into a pharmaceutically acceptable acid addition salt thereof.

In the above formulas, R is a hydrogen atom, a $C_{1-5}$ alkyl group, a heteroaryl group, a 2,1,3-benzoxadiazolyl group or a phenyl group which may be substituted by a substituent selected from the group consisting of a halogen atom, a $C_{1-4}$ alkyl group, a trifluoromethyl group, a $C_{1-4}$ alkoxy group, a nitro group and a cyano group; $R^1$ is a cyano group, a $C_{1-4}$ alkoxy-carbonyl group or a group represented by the formula of $-COOCH(R^5)CH_2R^6$ [wherein $R^5$ is a hydrogen atom or an aryl group and $R^6$ is a $C_{1-4}$ alkoxy group or a group represented by the formula of -N(Ra)(Rb) (wherein each of Ra and Rb is a $C_{1-4}$ alkyl group or an aralkyl group, or Ra and Rb together with the adjacent nitrogen atom form a heterocycle)]; $R^2$ is a $C_{1-4}$ alkyl group, a hydroxymethyl group, an acetoxymethyl group, a di-$C_{1-4}$ alkoxy-methyl group, a $C_{1-4}$ alkyl-carbamoyl-oxymethyl group or a hydroxyiminomethyl group; $R^3$ is a $C_{1-4}$ alkyl group, a formyl group, a carboxyl group, a nitro group or a group represented by the formula of $-N(R^7)(R^8)$ (wherein each of $R^7$ and $R^8$ is a hydrogen atom, a $C_{1-8}$ alkyl group, an aryl group, an aralkyl group or an acyl group, or $R^7$ and $R^8$ together with the adjacent nitrogen atom form a heterocycle), $-OR^9$ or $-SR^{10}$ (wherein each of $R^9$ and $R^{10}$ is a hydrogen atom, a $C_{1-8}$ alkyl group, an aralkyl group or an acyl group); $R^4$ is a hydrogen atom or a group represented by the formula of $-(CH_2)_mN(Rc)(Rd)$ (wherein each of Rc and Rd is a $C_{1-4}$ alkyl group or an aralkyl group, or Rc and Rd together with the

adjacent nitrogen atom form a heterocycle and m is an integer of from 1 to 3); n is 0 or an integer of from 1 to 3; and X is a reactive atom or group.

17. A pharmaceutical composition for the treatment of coronary and cerebral circulatory diseases comprising an effective amount of a compound of claim 1 and pharmaceutically acceptable additive.